# EUROPEAN PATENT APPLICATION

(11) **EP 1 138 672 A1**
(43) Date of publication of application: **04.10.2001**
(21) Application number: 01114294.0
(22) Date of filing: 08.01.1999
(51) Int. Cl.: C07D 207/14

(54) **Process for the preparation of 3-amino-pyrrolidine derivatives**

(30) Priority: 12.01.1998 EP 98100381
(62) Divisional of application: 99100213.0
(71) Applicant: Basilea Pharmaceutica AG, 4102 Binningen (CH)
(72) Inventor: Spurr, Paul, 4125 Riehen (CH); Wang, Shaoning, 4056 Basel (CH); Klinkhammer, Uwe, 79427 Eschbach (DE)
(74) Representative: Kjellsaa-Berger, Hanny, Dr.

(57) **Abstract**

The invention is concerned with a process for the manufacture of 3-amino-pyrrolidine derivatives of the formula 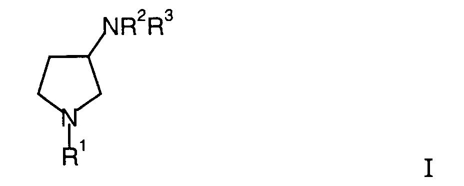 wherein
- R¹: signifies hydrogen, alkyl, cyclo-alkyl, alkenyl, aryl or an amino protecting group; and
- R², R³: each independently signify hydrogen, alkyl, cyclo-alkyl, alkenyl or aryl;
by converting a compound of the formula 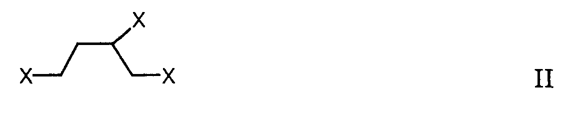 wherein
- X: signifies a protected hydroxy group;
in the presence of a primary amine of the formula R¹NH₂ into the pyrrolidine derivative of the formula 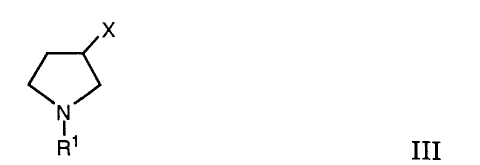 wherein X and R¹ have the aforementioned significances;
which is subsequently reacted in the presence of R²R³NH under pressure.

## Description

The present invention is concerned with a novel process for the manufacture of racemic and optically active 3-amino-pyrrolidine derivatives.

3-Amino-pyrrolidine derivatives, especially optically active 3-amino-pyrrolidine derivatives, are important intermediates for the production of agrochemicals and of pharmaceutically active substances such as, for example, of vinylpyrrolidinonecephalosporin derivatives.

3-Amino-pyrrolidine derivatives can be manufactured in a manner known per se, for example as described in EP-A 0 218 249 starting from 1,2,4-trisubstituted butane derivatives such as e.g. tribromobutane or trihydroxybutane. The racemic derivatives can then, if desired, can be converted by a racemate resolution into optically active 3-amino-pyrrolidine derivatives, JP 09124595-A. A process for the manufacture of optically active 3-amino-pyrrolidine derivatives based on the conversion of 4-hydroxy-proline as described, for example, in *J*. *Med. Chem.* 1764(92), 35, gives optically active 3-aminopyrrolidine over 3 steps.

The known methods for the manufacture of 3-aminopyrrolidine derivatives as described, for example, in UK Patent No. 1 392 194, EP 0 391 169 and US Patent No. 4 916 141, are time consuming and lead to expensive intermediates. The interest in other processes for the manufacture of 3-amino-pyrrolidine derivatives, especially of optically active 3-amino-pyrrolidine derivatives, is therefore extremely high. It has now been found that 3-amino-pyrrolidine derivatives, especially optically active 3-amino-pyrrolidine derivatives, can be manufactured in high yields in a simple manner from 1,2,4-trihydroxybutane derivatives.

The invention is accordingly concerned with a process for the manufacture of 3-amino-pyrrolidine derivatives of the formula 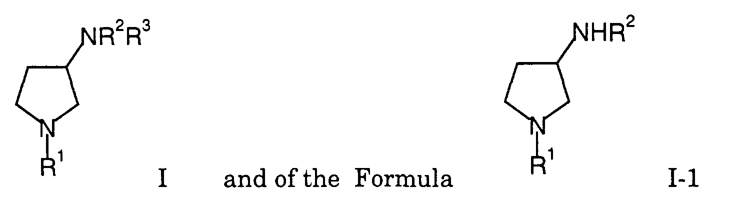 wherein
- R¹: signifies hydrogen, alkyl, cyclo-alkyl, alkenyl, aryl or an amino protecting group; and
- R², R³,: each independently signify hydrogen, alkyl, cyclo-alkyl, alkenyl or aryl;
which process comprises converting a compound of the formula 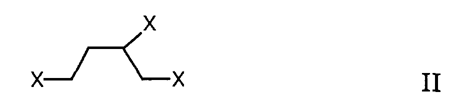 wherein
- X: signifies a protected hydroxy group;
in the presence of a primary amine of formula R¹NH₂ into the pyrrolidine derivative of the formula 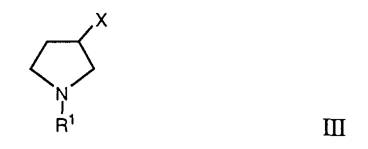 wherein X and R¹ have the aforementioned significances;
which is subsequently reacted in the presence of R²R³NH under pressure.

The term "protected hydroxy group" embraces in the scope of the present invention ester groups, for example, sulphonates such as mesylate, tosylate p-bromobenzenesulphonate or p-nitrobenzenesulphonate. These are especially groups which are cleaved off selectively under the reaction conditions for the ring closure in the presence of an amine of the formula NH₂R such that the protected hydroxy group X in the 2-position is not liberated. Mesylate and tosylate are especially preferred protected hydroxy groups X.

The term "amino protecting group" embraces in the scope of the present invention alkyl, benzyl, alkenyl, alkyloxycarbonyl, alkenyloxycarbonyl, benzyloxycarbonyl and the like. Allyl, benzyl, tert-butyloxycarbonyl, allyloxycarbonyl and benzyloxycarbonyl are especially preferred.

The term "alkyl" embraces in the scope of the present invention straight-chain and branched, optionally chiral hydrocarbon groups with 1 to 12, especially 1 to 8, carbon atoms such as, for example, methyl, ethyl, propyl, butyl, pentyl, hexyl, heptyl, octyl, i-propyl, i-butyl, tert-butyl, 2-methylbutyl, and the like.

The term "alkenyl" embraces in the scope of the present invention unsaturated, straight-chain and branched hydrocarbon groups with 3 to 12, especially 3 to 8 carbon atoms such as, for example, allyl, butenyl, pentenyl, and the like.

The term "cyclo-alkyl" embraces in the scope of the present invention cyclic hydrocarbon groups with 3 to 8 carbon atoms such as, for example, cyclo-propyl, cyclobutyl, cyclo-pentyl, cyclo-hexyl, cyclo-heptyl and cyclo-octyl.

The term "aryl" embraces in the scope of the present invention aromatic hydrocarbon groups optionally mono- or multiply-substituted with alkyl or halogen such as, for example, phenyl, tolyl and naphthyl, as well as aromatic 6-ring heterocycles such as, for example, pyridine, pyrimidine and pyridazine.

The process in accordance with the invention is especially suitable for the manufacture of optically active 3-amino-pyrrolidine derivatives of the formulae 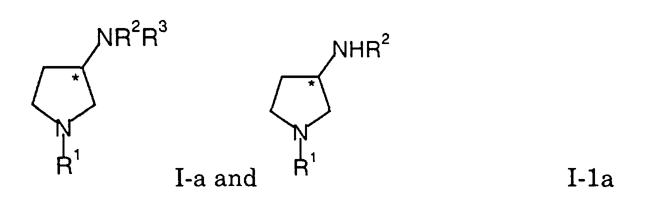 The process is distinguished over known processes primarily in that on the one hand by virtue of the use of a chiral educt of the formula 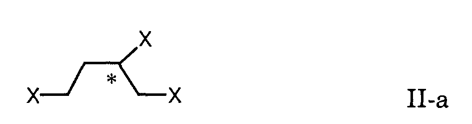 the racemate resolution is no longer necessary and on the other hand the number of steps for the manufacture of the 3-amino substituted pyrrolidine derivative of the formulae I-a and I-1a, especially 3-amino-pyrrolidine derivatives of formula I-a in which R² and R³ signify hydrogen which proceeds with high optical and chemical yields, is reduced by appropriate choice of the protecting groups.

In an especially preferred aspect of the process, optically active butyl-1,2,4-trimesylate (methanesulphonic acid 3-methanesulphonyloxy-1-methanesulphonyloxymethyl-propyl ester) is converted in the presence of a primary amine R¹ NH₂, wherein R¹ signifies benzyl, in tetrahydrofuran at a temperature of 0°C to 70°C, preferably at 50-60°C, into the corresponding optically active pyrrolidine derivative of formula III; the amino protecting group R¹ (benzyl) in the pyrrolidine derivative of formula III is replaced in the presence of allyl haloformate by allyloxycarbonyl in an inert solvent such as a hydrocarbon e.g. heptane, at temperatures of 0-100°C, preferably of 30-70°C; subsequently the desired optically active 3-amino-pyrrolidine derivative of formula I-a or 1-1a is obtained by introducing the amino group in the presence of R²R³NH, optionally in a solvent, such as e.g. tetrahydrofuran or dimethoxyethane, under pressure, preferably under a pressure of 3x10⁶ - 2x10⁷ PA, especially under a pressure of 5x10⁶ - 8x10⁶ PA, at a temperature of 20-200°C, preferably 100-150°C.

The process in accordance with the invention is especially suitable for the manufacture of optically active 1-allyloxy-3-amino-pyrrolidine, an intermediate for the production of vinylpyrrolidinone-cephalosporin derivatives of the formula 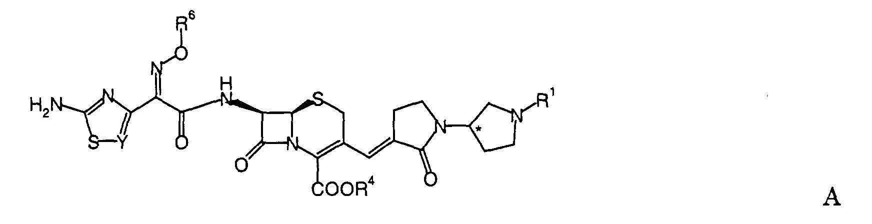 wherein
- Y: signifies CH or nitrogen;
- R⁶: signifies hydrogen or alkyl;
- R⁴: signifies hydrogen, an alkali metal ion or a tertiary ammonium group or an acid protecting group;
- R¹: denotes hydrogen, alkyl or an amino protecting group;
- *: denotes a centre of chirality.

Compounds of formula A are cephalosporin derivatives having a high antibacterial activity, especially against methicillin-resistant strains of Staphylococcus *aureus* (MRSA) and Pseudomonas *aeruginosa.*

Compounds of general formula A can be produced, for example, in a convergent synthesis as described in EP-A-0 849269 (based on EP-A-97119528.4) in accordance with Scheme I:

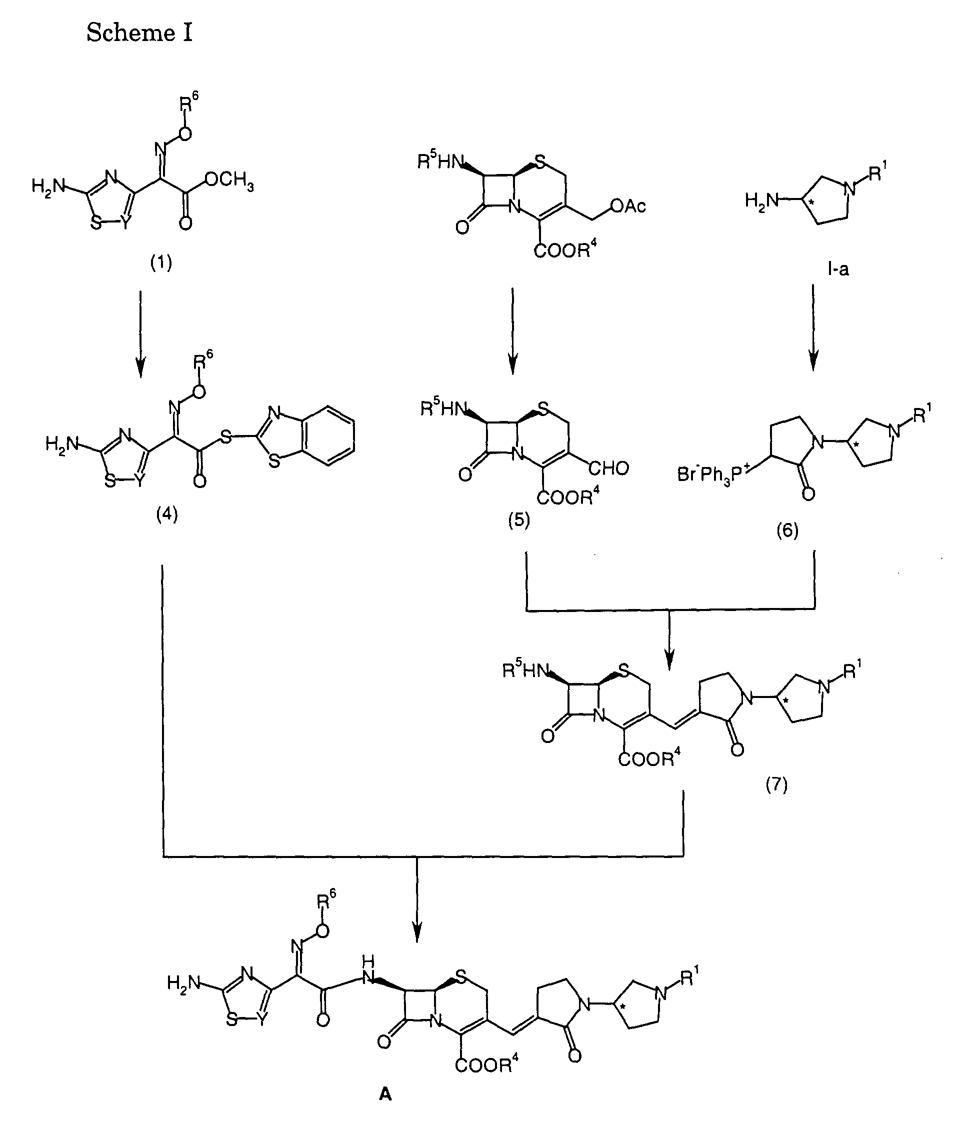

The symbols used in Scheme I have the aforementioned significances and R⁵ signifies an amino protecting group.

The 3-amino-pyrrolidine derivative of formula I-a in which R² and R³ signify hydrogen is manufactured in accordance with the invention according to the method described above, thereafter reacted with chloro-2-bromobutyroyl chloride and the N-substituted 3-bromopyrrolidone is converted into the Wittig salt (6) which is reacted in accordance with Scheme 1 with the cephemaldehyde (5). The substituted cephem derivative (7) is then reacted with an activated acyl derivative (4) to give the vinylpyrrolidinone-cephalosporin derivative of formula A.

The production of vinylpyrrolidinone-cephalosporin derivatives of formula A can be simplified by the use of the process in accordance with the invention, by which 3-amino-pyrrolidine derivatives of formula I-a and I-1a, especially 3-amino-pyrrolidine derivatives of formula I-a in which R² and R³ signify hydrogen, are accessible with high chemical and optical yields.

The present invention relates to the aspects described above and especially also to the use of the process for the manufacture of the compounds of formula I and of the compounds of formula A, which are manufactured according to the said processes.

The following Examples serve to further illustrate the invention.

### Example 1

### Preparation of methanesulphonic acid 3,4-bis-methanesulphonyloxy-butyl ester.

A solution of 18.1 ml of methanesulphonic acid in 45 ml of ethyl acetate is added dropwise within 2 hours under argon to a solution, cooled to 0-5°C, of 7.96 g of S-1,2,4-butanetriol and 33.5 ml of triethylamine in 90 ml of ethyl acetate. The white suspension was stirred at 0-5°C for a further 2 hours and thereafter the suspension was filtered and the yellow filtrate was washed in succession with 75 ml of 1N hydrochloric acid, 75 ml of saturated NaHCO₃ solution and 75 ml of saturated NaCl solution. The organic phase was dried over Na₂SO₄, filtered and the filtrate was concentrated. 21.79 g of yellow oil were obtained.

### Example 2

### Preparation of methanesulphonic aicd (S)-1-benzyl-pyrrolidin-3-yl ester.

a) 21.8 ml of benzylamine were added dropwise to a solution of 17 g of methanesulphonic acid 3,4-bis-methanesulphonyloxy-butyl ester (from Example 1) in 200 ml of tetrahydrofuran under argon, with the solution warming to 30°C. The solution was boiled at reflux for 24 hours, thereafter cooled to 0-5°C and the benzylammonium salt which thereby precipitated was filtered off. The filtrate was concentrated, the residude was taken up in 150 ml of tert-butyl methyl ether and the emulsion which thereby resulted was saturated with dry ice. After 5 minutes the white slurry was dissolved with 150 ml of water. The organic phase was separated and washed with 50 ml of saturated NaCl solution and with 30 ml of saturated NaCl solution. The washed solution was extracted with 100 ml of tert-butyl methyl ether and the organic phases were combined, dried with Na₂SO₄, filtered and the filtrate was concentrated. 12.1 g of yellow oil were obtained.
b) 13.8 g of sodium hydride were suspended in 250 ml of tetrahydrofuran under argon and cooled to 0-5°C. Within 1.5 hours there was added dropwise to this suspension a yellow solution of 88.6 g of N-benzyl-3-S-pyrrolidinol in 500 ml of tetrahydrofuran, the mixture was stirred at 0-5°C for 15 minutes and treated dropwise within 2 hours with a solution of 42.74 ml of mesyl choride in 250 ml of tetrahydrofuran. After stirring at 0-5°C for 1 hour the suspension was filtered over speedex, the filtrate was concentrated and the oily residue was taken up in 1000 ml of tert-butyl methyl ether and 280 ml of 3N sodium hydroxide solution. The phases were separated, the organic phase was washed with 250 ml of sodium hydroxide solution. The phases were separated, the organic phase was washed with 250 ml of sodium hydroxide solution and the wash solution was extracted with 500 ml of tert-butyl methyl ether. The organic phases were combined, dried over Na₂SO₄, filtered and the filtrate was concentrated. There were obtained 103.24 g of brown oil which was purified by chromatography. 51.44 g of product were obtained.

### Example 3

### Preparation of allyl-3-methanesulphonyloxy-pyrrolidine- 1-carboxylate.

6.4 ml of allyl chloroformate were added dropwise to 10.2 g of methanesulphonic acid (S)-1-benzyl-pyrrolidin-3-yl ester in 880 ml of n-heptane at room temperature within 10 minutes under argon and with slight cooling. The two-phase mixture was stirred vigorously for 2 hours 45 minutes and subsequently treated with 40 ml of methanol/water (1:1). The aqueous methanolic phase was separated, extracted with 40 ml of heptane and thereafter the methanol was distilled off on a rotary evaporator. The aqueous phase was extracted twice with 30 ml of ethyl acetate. The organic phases were combined, dried over Na₂,SO₄, filtered and the filtrate was concentrated. 8.23 g of beige oil were obtained.

### Example 4

### Preparation of allyl 3-amino-pyrrolidine-1-carboxylate.

1 g of allyl 3-methanesulphonyloxy-pyrrolidine-1-carboxylate was placed in an autoclave, evacuated 4 times under argon and again exposed to air and subsequently cooled in an acetone/CO₂ bath. The reaction was effected at 8×10⁶ PA with ammonia at a temperature of 110°C. After stirring for 90 minutes the autoclave was cooled, the residue was taken up in methylene chloride, the suspension was filtered and the filtrate was concentrated. There was obtained 0.68 g of pale brown oil with an e.e. (enantiomeric excess) of 96.7%.

### Example 5

### Preparation of 1-benzyl-3-amino-pyrrolidine.

1.94 g of methanesulphonic acid (S)-1-benzyl-pyrrolidin-3-yl ester were placed in an autoclave, evacuated 4 times under argon and again exposed to air, and then cooled in an acetone CO₂ bath. The reaction was effected at 8×10⁶ PA with ammonia at a temperature of 110°C. After stirring for 150 minutes the autoclave was cooled, the residue was taken up in methylene chloride, the suspension was filtered and the filtrate was concentrated. 1.33 g of pale brown oil were obtained. After purification by chromatography there were obtained 1.26 g of product with an e.e. (enantiomeric excess) of 96.8%.

### Example 6

### Preparation of benzyl (R)-3-amino-pyrrolidine-1-carboxylate.

### a) Preparation of benzyl (S)-3-hydroxy-pyrrolidine-1-carboxylate.

The pH of a solution of 6.18 g of (S)-3-hydroxy-pyrrolidine hydrochloride in 175 ml of water was adjusted to 10 with 10% sodium hydroxide solution and cooled to 0°-5°C. 7.1 ml of benzyl chloroformate were added dropwise within 30 minutes under argon, with the pH of the solution being held between 9.5 and 11.5 by the dropwise addition of 10% sodium hydroxide solution. After completion of the addition the suspension was stirred at room temperature for 16 hours. The suspension was extracted with ethyl acetate, the organic phase was washed with water, dried over Na₂SO₄, filtered and the filtrate was concentrated. Purification of the crude product over a silica gel column gave 7.33 g of benzyl (R)-3-hydroxy-pyrrolidine-1-carboxylate as a beige liquid.

### b) Preparation of benzyl (S)-3-methanesulphonyloxy-pyrrolidine-1-carboxylate.

A solution of 7.3 g of benzyl (R)-3-hydroxy-pyrrolidine-1-carboxylate and 5.56 ml of triethylamine in 80 ml of ethyl acetate was cooled to 0°-5°C under argon and treated within 30 minutes with a solution of 2.97 ml of mesyl chloride in 20 ml of ethyl acetate. After stirring at room temperature for 2 hours and leaving to stand for 16 hours the suspension was diluted with 40 ml of water, stirred for 10 minutes and the organic phase was separated. The organic phase was extracted in succession with 20 ml of 1N HCl, 20 ml of saturated NaHCO₃ solution and 10 ml of saturated NaCl solution. The organic phase was dried over Na₂SO₄, filtered and the filtrate was concentrated to give 10.18 g of product as a beige oil.

### c) Preparation of benzyl (R)-3-amino-pyrrolidine-1-carboxylate

5.0 g of benzyl (S)-3-methanesulphonyloxy-pyrrolidine-1-carboxylate were placed in an autoclave, evacuated four times under argon and cooled in an acetone/CO₂ bath. After the addition of ammonia the reaction was carried out at 1,36×10⁷ PA at a temperature of 150°C. After stirring for 40 minutes the autoclave was cooled, the residue was taken up in methylene chloride, the suspension was filtered and the filtrate was concentrated. There were obtained 3.52 g of pale yellow oil, e.e. 97%.

### Example 7

### Preparation of tert-butyl (R)-3-amino-pyrrolidine-1-carboxylate.

### a) Preparation of tert-butyl (S)-3-hydroxy-pyrrolidine-1-carboxylate.

34.1 g of (S)-3-hydroxy-pyrrolidinol hydrochloride and 29.2 g of K₂CO₃ were suspended in 400 ml of methanol under argon. The suspension was cooled to 0°-5°C and treated while stirring within 10 minutes with 45.8 g of di-tert-butyl dicarbonate. The reaction mixture was stirred firstly at 0-5°C for 30 minutes and subsequently at room temperature for 4.5 hours. The suspension was concentrated and the residue was taken up in 400 ml of ethyl acetate and 200 ml of water. The organic was separated, washed with water, dried over Na₂SO₄, filtered and the filtrate was concentrated. There were obtained 34.1 g of product as a brownish liquid, which was used in the next step without purification.

### b) Preparation of methanesulphonic acid (S)-1-carboxylic acid tert-butyl-pyrrolidin-3-yl ester

34.1 g of tert-butyl (S)-3-hydroxy-pyrrolidine-1-carboxylate and 29.2 ml of triethylamine were dissolved in 300 ml of ethyl acetate under argon and cooled to 0°-5°C before the dropwise addition within 30 minutes of 3.91 ml of mesyl chloride in 20 ml of ethyl acetate. The resulting suspension was stirred at 0°-5°C for 1.5 hours and subsequently at room temperature for 16 hours. The suspension was diluted with 150 ml of water, stirred for 10 minutes and the organic phase was separated. The organic phase was washed in succession with 1N HCl, saturated NaHCO₃ solution and saturated NaCl solution. The organic phase was dried over Na₂SO₄ and filtered. The filtrate was concentrated and gave 50.4 g of product as a beige liquid.

### c) Preparation of tert-butyl (R)-3-amino-pyrrolidine-1-carboxylate

5.0 g of methanesulphonic acid (S)-1-carboxylic acid tert-butyl-pyrrolidin-3-yl ester were placed in an autoclave, evacuated four times under argon and cooled in an acetone/CO₂. After the addition of ammonia the reaction was carried at 1,32x10⁷ PA at a temperature of 150°C. After stirring for 2 hours the autoclave was cooled, the residue was taken up in methylene chloride, the suspension was filtered and the filtrate was concentrated. There were obtained 3.32 g of pale yellow oil, e.e. 97%.

## Claims

1. A process for the manufacture of 3-amino-pyrrolidine derivatives of the formula 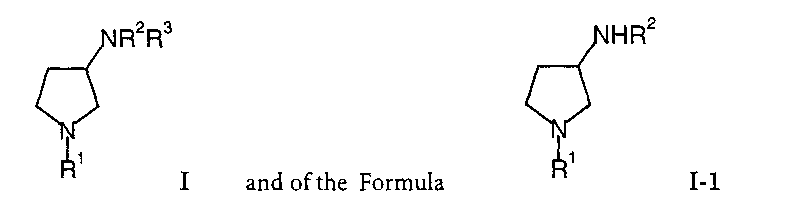 wherein
R¹ signifies hydrogen, alkyl, cyclo-alkyl, alkenyl, aryl or an amino protecting group; and
R², R³ independently signify hydrogen, alkyl, cyclo-alkyl, alkenyl or aryl;
which process comprises converting a compound of the formula 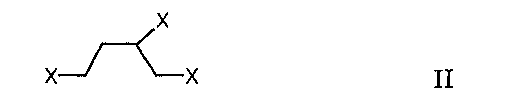 wherein
X signifies a protected hydroxy group;
in the presence of a primary amine of the formula R¹NH₂ into the pyrrolidine derivative of the formula 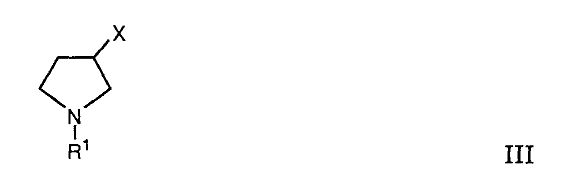 wherein X and R¹ have the aforementioned significances;
which is subsequently reacted in the presence of R²R³NH under pressure and optionally in a solvent.

2. A process according to claim 1 for the manufacture of compounds of the formula 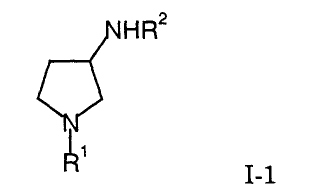 wherein R¹ and R² have the significances give in claim 1.

3. A process according to claim 1 or 2, wherein the reaction with R²R³NH or R²NH₂ is carried out at a temperature of 20-200°C under a pressure of 3x10⁶ - 2x10⁷ PA, preferably 5x10⁶ - 8x10⁶ PA.
